# NEUE EUROPÄISCHE PATENTSCHRIFT

(11) **EP 0 292 669 B2**
(45) Veröffentlichungstag und Bekanntmachung des Hinweises auf die Entscheidung über den Einspruch: **16.03.1994**
(45) Hinweis auf die Patenterteilung: 04.12.1991
(21) Anmeldenummer: 88104912.6
(22) Anmeldetag: 26.03.1988
(51) Int. Cl.: B60R 21/32

(54) **Aufprallsensor für Kraftfahrzeuge**
Vehicle impact sensor
Détecteur de collision pour les véhicules automobiles

(30) Priorität: 23.05.1987 DE 3717427
(43) Veröffentlichungstag der Anmeldung: 30.11.1988
(73) Patentinhaber: Deutsche Aerospace AG, 81663 München (DE)
(72) Erfinder: Wöhrl, Alfons, D-8898 Schrobenhausen (DE); Hora, Peter, D-8898 Schrobenhausen (DE)

(56) Entgegenhaltungen:
- DE-A- 2 123 359
- DE-A- 2 151 399
- DE-A- 2 920 147
- US-A- 3 701 903
- US-A- 4 021 057
- US-A- 4 166 641

## Beschreibung

Die Erfindung bezieht sich auf einen Aufprallsensor für Kraftfahrzeuge oder dergleichen gemäß dem Oberbegriff des Patentanspruches 1, der aus der DE-A-2920147 abgeleitet wurde.

Ein Solcher Aufprallsensor für die Auslösung einer passiven Sicherheitseinrichtung zum Schutze der Insassen eines Kraftfahrzeuges ist aus der DE-A-22 07 831 bekannt. Dieser Aufprallsensor weist einen Beschleunigungsaufnehmer, z.B. einen piezoelektrischen Kristall auf, dessen Empfindlichkeitsachse in einer im wesentlichen horizontalen Ebene in Fahrtrichtung angeordnet ist. Die Ausgangssignale des Piezokristalls werden einer Auswerteschaltung zugeführt. in der die Ausgangssignale nach einer Schwellenbewertung im wesentlichen einfach oder doppelt integriert werden. Übersteigt bei einem Aufprall des Kraftfahrzeuges das ausgewertete Signal einen bestimmten Schwellenwert entsprechend einer kritischen Geschwindigkeit bei einer einfachen Integration oder einem kritischen Weg bei einer doppelten Integration, so wird ein Auslösesignal an die passive Sicherheitseinrichtung abgegeben und damit diese aktiviert. Die passive Sicherheitseinrichtung ist z.B. ein aufblasbares Luftkissen oder eine Gurtstrammereinrichtung zum Strammen bzw. Festziehen der Sicherheitsgurte für Kraftfahrzeuginsassen.

Aus der DE-A-21 51 399 ist eine stoßempfindliche Anordnung mit wenigstens einem piezoelektrischen Kristall als mechanisch-elektrischer Umformer bekannt.Bei dieser Anordnung soll nur zwischen kurz anhaltenden und über längere Zeit andauernden Stößen (Zusammenstößen) unterschieden werden. An eine Winkelerfassung des Aufpralls ist dort nicht gedacht.

Neben der Verwendung von passiven Sicherheitseinrichtungen sind heutige Kraftfahrzeuge auch mit sogenannten aktiven Sicherheitseinrichtungen versehen, die insbesondere dadurch erzielt werden, daß die Fahrzeugkarossierie sogenannte Knauschzonen aufweist, die sich bei einem Aufprall des Fahrzeuges in vorgegebenem Maße deformieren und einen Großteil der Aufprallenergie bereits absorbieren. Durch die konstruktive Auslegung der Knautschzonen ist jedoch das Risiko gegeben, daß die von dem Beschleunigungsaufnehmer abgegebenen Ausgangssignale sozusagen verzögert reagieren und die passive Sicherheitseinrichtung dementsprechend verspätet ausgelöst wird. Wünschenswert wäre ein Aufprallsensor, der sicherstellt. daß die aktiven und passiven Sicherheitseinrichtungen in optimaler Weise zusammenarbeiten.

Dementsprechend liegt der Erfindung die Aufgabe zugrunde, einen Aufprallsensor der in Rede stehenden Art anzugeben, der in einfacher Weise an die konstruktive Ausbildung der Knautschzonen individueller Fahrzeuge angepaßt werden kann sowie eine sichere, folgerichtige und rechtzeitige Auslösung der passiven Sicherheitseinrichtung gewährleistet; das beinhaltet auch die Fähigkeit unter allen möglichen Fahrwinkeln zwischen Aufprall von vorne und Aufprall von hinten unterscheiden zu können.

Diese Aufgabe ist gemäß der Erfindung durch die im kennzeichnenden Teil des Patentanspruches 1 angegebenen Merkmale gelöst.

Demgemäß weist der Aufprallsensor mehrere. im allgemeinen zwei Beschleunigungsaufnehmer mit gerichteten Empfindlichkeitsachsen auf, die entsprechend der konstruktiven Ausbildung der Knautschzonen im Kraftfahrzeug oder dergleichen unter einem bestimmten Winkel zueinander angeordnet sind. Für jeden Beschleunigungsaufnehmer ist innerhalb der Auswerteschaltung ein eigener Kanal für die Signalverarbeitung vorgesehen, wobei eine Bewertungsschaltung in Verbindung mit einer Logik dafür sorgt, daß die Ausgangssignale der Beschleunigungsaufnehmer so ausgewertet werden, daß ein Auslösesignal nur dann erfolgt, wenn am Ausgang mindestens einer Auswerteschaltung ein Signal größer als eine erste große Schwellwertspannung und gleichzeitig am Ausgang mindestens einer weiteren Auswerteschaltung, bezogen auf die Fahrtrichtung des Fahrzeuges mit einer Winkellage anderen Vorzeichens, ein Signal größer einer zweiten kleinen Schwellwertspannung anliegt.

Mit dieser Auslegung kann die spezifische winkelabhängige Struktur des jeweiligen Kraftfahrzeuges oder dergleichen bei einem Aufprall berücksichtigt werden. Es kann damit auch unter allen möglichen Fahrwinkeln zwischen Aufprall von vorne und Aufprall von hinten unterschieden werden. Die Aufnehmer werden vorzugsweise so eingebaut, daß ihre Empfindlichkeitsachsen gegenüber der Fahrtrichtung des Kraftfahrzeuges jeweils einen spitzen Winkel einnehmen. Diese Winkel können gleich, aber auch ungleich sein. In vielen Fällen hat es sich als vorteilhaft erwiesen zwei Beschleunigungsaufnehmer zu verwenden, deren Empfindlichkeitsachsen + 30° bzw. -30° in bezug zu der Fahrtrichtung ausgerichtet sind. Andere Anordnungen mit z.B. +30° und -45° können für spezielle konstruktive Ausbildungen der Knautschzonen des Fahrzeuges vorteilhaft sein.

Die Signalverarbeitung erfolgt in dem Aufprallsensor entweder analog, digital oder kombiniert analog und digital.

Die Bewertung der Ausgangssignale der Beschleunigungsaufnehmer kann in einfacher Weise durch Differenzverstärkerstufen in den Kanälen zur Signalverarbeitung erfolgen, mit denen die Ausgangssignale mit Schwellwerten verglichen werden. Ein solcher Schwellwert kann z.B. ein minimaler kritischer Geschwindigkeitswert sein. Sobald dieser Schwellwert in einem Kanal erreicht wird und ein zweiter kleinerer Referenzwert im anderen Kanal ebenfalls überschritten ist, wird ein Ausgangssignal erzeugt. Durch diese Verknüpfung wird erreicht, daß beide Signalverarbeitungskanäle einen Referenzwert überschreiten müssen, bevor der Kanal mit der größeren Signalamplitude das Ausgangssignal erzeugt.

Bei einem Heck- bzw. Seitenaufprall wird in beiden (Heckaufprall) oder ein einem der beiden Signalverarbeitungskanäle (Seitenaufprall) der Referenzwert nicht überschritten, es erfolgt kein Ausgangssignal.

Im Gegensatz zu früheren Untersuchungen hat es sich herausgestellt, daß es bei einer Anordnung der Beschleunigungsaufnehmer gemäß der Erfindung nicht notwendig ist, einen Beschleunigungsaufnehmer mit seiner Empfindlichkeitsachse in Fahrtrichtung des Fahrzeuges oder dergleichen auszurichten. Vielmehr ist es für die rechtzeitige Auslösung der passiven Sicherheitseinrichtung vorteilhaft, gerade von dieser bisher gültigen Lehre abzuweichen und die Empfindlichkeitsachsen der verwendeten Beschleunigungsaufnehmer mit einem mehr oder minder spitzen Winkel gegen die Fahrtrichtung des Kraftfahrzeuges anzustellen.

Durch diese Anordnung der Beschleunigungsaufnehmer wird eine Auswertung in der Ebene vorgenommen, wobei über die Empfindlichkeitscharakteristik der beiden Beschleunigungsaufnehmer eindeutig die Richtung des Aufpralles detektiert werden kann.

Auslösungen bei Heckaufprällen im Winkelbereich 180° ± 90° werden durch die Verknüpfung der Komparatoren 13 bis 16 unterdrückt. Dies erfolgt dadurch, daß bei einem Seiten- bzw. Heckaufprall die Komparatoren 14 bzw. 16 ein Low-Signal liefern, so daß eine Durchschaltung der Signale der Komparatoren 13 bzw. 15 verhindert wird.

Weitere Ausgestaltungen der Erfindung gehen aus den Unteransprüchen hervor.

Die Erfindung ist in einem Ausführungsbeispiel anhand der Zeichnung näher erläutert. In dieser stellen dar:
- Figur 1: schematisch den Einbau zweier Beschleunigungsaufnehmer mit unterschiedlich gerichteten Empfindlichkeitsachsen in einem Kraftfahrzeug;
- Figur 2: ein Blockschaltbild eines Aufprallsensors gemäß der Erfindung mit zwei gemäß Figur 1 ausgerichteten Beschleunigungsaufnehmern in einem ersten Ausführungsbeispiel und
- Figur 3: ein Blockschaltbild eines Aufprallsensors mit zwei gemäß Figur 1 ausgerichteten Beschleunigungsaufnehmern gemäß einem zweiten Ausführungsbeispiel der Erfindung.

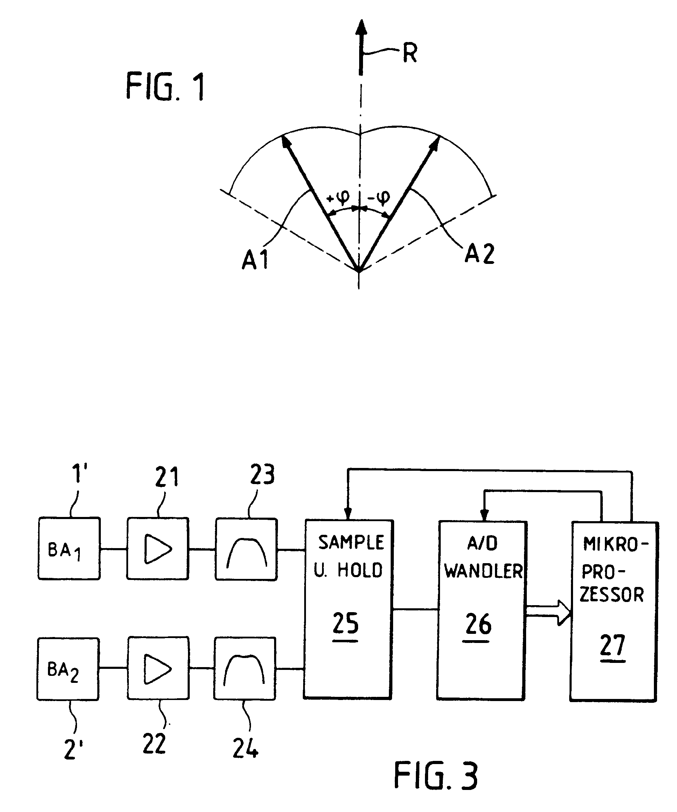

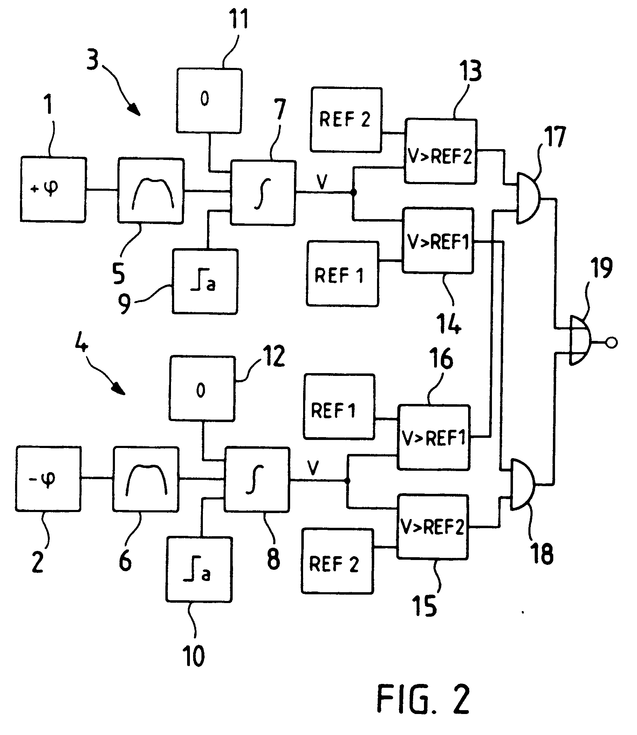

In Figur 2 ist ein Blockschaltbild für einen Aufprallsensor dargstellt, der zwei Beschleunigungsaufnehmer 1 und 2 aufweist, die herkömmlicher Bauart sein können. Die Beschleunigungsaufnehmer haben jeweils eine gerichtete Empfindlichkeitsachse, die in Figur 1 mit A1 bzw. A2 bezeichnet ist. Wie aus dieser Figur hervorgeht, sind die Empfindlichkeitsachsen mit einem Winkel + φ bzw. - φ gegen die durch einen Pfeil R bezeichnete Fahrtrichtung eines Kraftfahrzeuges angestellt, in diesem Falle jeweils um den Winkel ± 30°. Die Beschleunigungsaufnehmer 1 und 2 sprechen jeweils in einem Bereich um diese Empfindlichkeitsachsen entsprechend einer cos φ -Charakteristik an, der in Figur 1 durch die beiden Sektoren gekennzeichnet ist.

Die Ausgangssignale der beiden Beschleunigungsaufnehmer 1 und 2 werden in gleich aufgebauten Signalkanälen 3 bzw. 4 ausgewertet.

Das Ausgangssignal jedes Beschleunigungsaufnehmers wird zunächst einer Vorfilterstufe 5 bzw. 6 und anschließend einem Integrator 7 bzw. 8 zugeführt. Für jeden Integrator ist eine Schwellenschaltung 9 bzw. 10 vorgesehen, die jeweils eine Schwelle a festlegen, oberhalb der das von den Signalformerstufen 5 bzw. 6 abgegebene Signal aufintegriert wird. Für jeden Integrator ist zusätzlich noch ein Null-Steller 11 bzw. 12 vorgesehen. Da das von jeder Signalformerstufe 5 bzw. 6 abgegebene Signal von der Beschleunigung bzw. Verzögerung abhängig ist, die auf den entsprechenden Beschleunigungsaufnehmer 1 bzw. 2 wirkt, ist das Ausgangssignal der Integratoren 7 und 8 ein geschwindigkeitsabhängiges Signal V.

Dieses geschwindigkeitsabhängige Signal wird jeweils zwei Komparatoren 13 und 14 bzw. 15 und 16 zugeführt, in denen es mit Schwellwerten verglichen wird. Diese Schwellwerte REF1 und REF2 werden jeweils an die zweiten Eingänge der Komparatoren 14 bzw. 13 sowie 16 bzw. 15 gelegt. Der Schwellwert REF2 ist größer als der Schwellwert REF1. Diese Schwellwerte entsprechen kritischen Geschwindigkeiten, bei denen eine Auslösung der passiven Sicherheitseinrichtung, z.B. eines Airbag erfolgen sollte. Sie werden anhand von Versuchswerten festgelegt und können je nach Fahrzeugtyp unterschiedlich sein. In den Komparatoren 14 und 16 werden die Ausgangssignale V der Integratoren 7 bzw. 8 mit dem Schwellwert REF1 verglichen, in den Komparatoren 13 bzw. 15 die Ausgangssignale V der Integratoren 7 bzw. 8 mit dem Schwellwert REF2. Das Ausgangssignal des Komparators 13 für den Signalkanal des Beschleunigungsaufnehmers 1 und das Ausgangssignal des Komparators 16 im Signalkanal für den Beschleunigungsaufnehmer 2 werden einem UND-Gatter 17 zugeführt. Entsprechend werden die Ausgänge der Komparatoren 14 und 15 mit einem zweiten UND-Gatter 18 verbunden. Die Ausgänge beider UND-Gatter 17 und 18 sind mit einem ODER-Gatter 19 verbunden, dessen Ausgang mit einer Auslöseschaltung für die passive Sicherheitseinrichtung verbunden ist.

In dem beschriebenen Fall wird durch die Verknüpfung der Komparatoren 13 bis 16 über die UND-Glieder 17 und 18 die passive Sicherheitseinrichtung in Abhängigkeit der Signale der Beschleunigungsaufnehmer ausgelöst. Durch die räumliche Anordnung der Emfindlichkeitsachsen der Beschleunigungsaufnehmer und durch diese Verknüpfung wird somit die passive Sicherheitseinrichtung nur bei Frontalaufprall im Winkelbereich von 0° bis ± 90° und zum frühest möglichen und damit für die Fahrzeuginsassen zum günstigsten Zeitpunkt ausgelöst.

In Figur 3 ist eine zweite Ausführungsform eines Aufprallsensors ebenfalls mit zwei Beschleunigungsaufnehmern 1' und 2' angegeben, die entsprechend Figur 1 im Kraftfahrzeug ausgerichtet sind. Die Ausgangssignale der Beschleunigungsaufnehmer 1' und 2' werden jeweils einem Vorverstärker 21 bzw. 22 zugeführt, deren Ausgänge mit einer Vorfilterstufe 23 bzw. 24 verbunden sind. Die Ausgangssignale dieser Vorfilterstufen werden in einer Abtast- und Halteschaltung 25 aufgenommen und gespeichert und anschließend einem Analog/Digitalwandler 26 zugeführt. Die digitalisierten Ausgangssignale des Analog/Digitalwandlers werden von einem Mikroprozessor 27 verarbeitet, der gleichzeitig die Funktion der Abtast- und Halteschaltung 25 sowie des Analog/Digitalwandlers 26 steuert. In dem Mikroprozessor 27 wird die Signalverarbeitung vergleichbar gemäß Figur 2 durchgeführt.

## Patentansprüche

1. Aufprallsensor für Kraftfahrzeuge oder dergleichen mit einer Beschleunigungsaufnehmeranordnung mit zwei oder mehreren Beschleunigungsaufnehmern (1, 2; 1', 2') mit gerichteten Empfindlichkeitsachsen (A1, A2), wobei die Empfindlichkeitsachsen (A1, A2) in unterschiedlichen Richtungen ausgerichtet sind, und mit einer für die Beschleunigungsaufnehmer (1, 2; 1', 2') vorgesehenen Bewertungsschaltung (13 bis 19; 27) hinsichtlich der Amplituden der ausgewerteten Ausgangssignale der Beschleunigungsaufnehmer (1, 2; 1', 2') und einer Auswerteschaltung (5 bis 12) für die Ausgangssignale der Beschleunigungsaufnehmeranordnung, wobei die Auswerteschaltung ein Auslösesignal an eine passive Sicherheitseinrichtung , z.B. ein aufblasbares Luftkissen, eine Einrichtung zum Strammen von Sicherheitsgurten oder dergleichen abgibt, wenn die Ausgangssignale der Beschleunigungsaufnehmeranordnung einen kritischen Schwellenwert übersteigen,
die passive Sicherheitseinrichtung aufgrund des Ausgangssignals der Auswerteschaltungen (5, 7, 9, 11 sowie 6, 8, 10, 12) aber nur dann auslöst, wenn am Ausgang mindestens einer Auswerteschaltung (5, 7, 9, 11) ein Signal größer als eine erste große Schwellwertspannung (13) und gleichzeitig am Ausgang einer weiteren Auswerteschaltung (6, 8, 10, 12), bezogen auf die Fahrtrichtung des Fahrzeugs mit einer Winkellage anderen Vorzeichens, ein Signal größer als eine zweite kleinere Schwellwertspannung (16) anliegt,
dadurch gekennzeichnet, daß
den Beschleunigungsaufnehmern (1, 2) jeweils eine Integratoranordnung (7, 8) nachgeschaltet ist, daß deren Ausgang mit jeweils mehreren Komparatoren (13 bis 16) verbunden ist, in denen das jeweilige Ausgangssignal der Integratoranordnungen (7, 8) mit mehreren Referenzwerten (REF1, REF2) verglichen wird, und daß die Ausgänge der Komparatoren mit einer logischen Verknüpfungsschaltung (17 bis 19) verbunden sind, wobei das Ausgangssignal des Komparators (13) für den Signalkanal des Beschleunigungsaufnehmers (1) und das Ausgangssignal des Komparators (16) im Signalkanal für den Beschleunigungsaufnehmer (2) einem UND-Gatter (17) zugeführt wird, die Ausgänge der Komparatoren (14, 15) mit einem zweiten UND-Gatter (18) entsprechend verbunden werden und die Ausgänge beider UND-Gatter (17, 18) mit einem ODER-Gatter (19) verbunden sind, dessen Ausgang mit einer Auslöseschaltung für die passive Sicherheitseinrichtung verbunden ist.

2. Aufprallsensor nach Anspruch 1, dadurch gekennzeichnet, daß der Aufprallsensor zwei Beschleunigungsaufnehmer (1, 2; 1', 2') aufweist. deren Empfindlichkeitsachsen (A1, A2) in bezug zu der Fahrtrichtung (R) des Kraftfahrzeuges oder dergleichen einen spitzen Winkel (φ) einnehmen.

3. Aufprallsensor nach Anspruch 2, dadurch gekennzeichnet, daß die Winkel der Empfindlichkeitsachsen (A1, A2) in bezug zuder Fahrrichtung (R) in einer gemeinsamen Ebene angeordnet sind.

4. Aufprallsensor nach Anspruch 2 oder 3, dadurch gekennzeichnet, daß die Winkel der Empfindlichkeitsachsen (A1, A2) in bezug zu der Fahrtrichtung (R) entgegengesetzt gleich sind.

5. Aufprallsensor nach einem der Ansprüche 1 bis 4, dadurch gekennzeichnet. daß die Bewertungsschaltung (25 bis 27) für die Ausgangssignale der Beschleunigungsaufnehmer (1', 2') einen Mikroprozessor (27) aufweist

6. Aufprallsensor nach Anspruch 2, dadurch gekennzeichnet, daß die Winkel der Empfindlichkeitsachsen (A1, A2,...) in bezug zu der Fahrtrichtung (R) nicht in einer gemeinsamen Ebene angeordnet sind.

## Claims

1. Impact sensor for motor vehicles or the like, comprising an acceleration-detector arrangement with two or more acceleration detectors (1, 2; 1', 2') with directional sensitivity axes (A1, A2), in which respect the sensitivity axes (A1, A2) are oriented in different directions, and with an evaluation circuit (13 to 19; 27) for the acceleration detectors (1, 2; 1', 2') relative to the amplitudes of the evaluated output signals of the acceleration detectors (1, 2; 1', 2'), and an evaluation circuit (5 to 12) for the output signals of the acceleration-detector arrangement, and that the evaluation circuit transmits a trigger signal to a passive safety system, for example an inflatable airbag, a device for tightening of safety belts or the like, when the output signals of the acceleration-detector arrangement exceed a critical threshold value, whilst the passive safety device triggers due to the output signal of the evaluation circuits (5, 7, 9, 11 as well as 6, 8, 10, 12) only if at the output of at least one evaluation circuit (5, 7, 9, 11) is a signal which is larger than a first large threshold signal voltage (13) and simultaneously a signal at the output of an additional evaluation circuit (6, 8, 10, 12), relative to the travel direction of the vehicle with an angular position of another sign, is larger than a second lower threshold voltage (16), **characterised in that** the acceleration detectors (1, 2) have at their output an integrator arrangement (7, 8), that their output is connected to a plurality of respective comparators (13 to 16) where the respective output signal of the integrator arrangements (7, 8) is compared to a plurality of reference values (REF1, REF2), and that the outputs of the comparators are connected to a logic connection circuit (17 to 19) and that the output signal of the comparator (13) for the signal channel of the acceleration detector (1) and the output signal of the comparator (16) in the signal channel for the acceleration detector (2) are fed to an AND-circuit (17), that the outputs of the comparators (14, 15) are accordingly connected to a second AND-circuit (18) and the outputs of both AND-circuits (17, 18) are connected to an OR-circuit (19) the output of which is connected to a trigger circuit for the passive safety device.

2. Impact sensor according to claim 1, **characteri****sed in that** the impact sensor comprises two acceleration detectors (1, 2; 1', 2'), the sensitivity axes of which (A1, A2) relative to the travel direction (R) of the motor vehicle or the like are at an acute angle (Φ).

3. Impact sensor according to claim 2, **characteri****sed in that** the angles of the sensitivity axes (A1, A2) are arranged in a common plane relative to the travel direction (R).

4. Impact sensor according to claim 2 or 3, **charac****terised in that** the angles of the sensitivity axes (A1, A2) relative to the travel direction (R) are opposing and equal.

5. Impact sensor according to one of the claims 1 to 4, **characterised in that** the evaluation circuit (25 to 27) for the output signals of the acceleration detector (1', 2') comprises a microprocessor (27).

6. Impact sensor according to claim 2, **characteri****sed in that** the angles of the sensitivity axes (A1, A2...) relative to the travel direction (R) are not arranged in a common plane.

## Revendications

1. Détecteur de collision pour véhicule automobile ou analogue comprenant un système de capteurs d'accélération comportant deux ou plusieurs capteurs d'accélération (1, 2; 1', 2') dont les axes de détection (A1, A2) sont orientés, les axes de détection (A1, A2) étant orientés dans des directions différentes, un circuit d'évaluation (13 à 19; 27) étant prévu pour les capteurs d'accélération (1, 2; 1', 2') pour l'évaluation des signaux de sortie des capteurs d'accélération (1, 2; 1', 2') du point de vue de leur amplitude, ainsi qu'un circuit d'exploitation (5 à 12) des signaux de sortie du système de capteurs d'accélération, le circuit d'exploitation délivrant un signal de déclenchement à un dispositif de sécurité passive, par exemple un coussin à air gonflable, un dispositif pour tendre les ceintures de sécurité ou analogue, lorsque les signaux de sortie du système de capteurs d'accélération dépassent un seuil critique, le dispositif de sécurité passive déclenchant à la suite du signal de sortie des circuits d'exploitation (5, 7, 9, 11 et 6, 8, 10, 12) uniquement lorsqu'apparaît à la sortie d'au moins un circuit d'exploitation (5, 7, 9, 11), un signal supérieur à une première tension de seuil (13), et simultanément à la sortie d'au moins un autre circuit d'exploitation (6, 8, 10, 12), avec une position angulaire de signe différent par rapport à la direction de déplacement du véhicule, un signal supérieur à une deuxième tension de seuil (16), caractérisé en ce qu'un dispositif d'intégration (7, 8) est monté derrière chacun des capteurs d'accélération (1, 2), en ce que leur sortie est reliée chaque fois à plusieurs comparateurs (13 à 16) dans lesquels le signal de sortie de chacun des dispositifs d'intégration (7, 8) est comparé à plusieurs valeurs de référence (REF1, REF2) et en ce que les sorties des comparateurs sont reliées à un circuit combinatoire logique (17 à 19), le signal de sortie du comparateur (13) dans le canal de signal du capteur d'accélération (1) et le signal de sortie du comparateur (16) dans le canal de signal du capteur d'accélération (2) étant transmis à une porte ET (17), les sorties des comparateurs (14, 15) étant reliées à une deuxième porte ET (18) et les sorties des deux portes ET (17, 18) étant reliées à une porte OU (19) dont la sortie est connectée à un circuit de déclenchement du dispositif de sécurité passive.

2. Détecteur de collision selon la revendication 1 caractérisé en ce que le détecteur de collision comporte deux capteurs d'accélération (1, 2; 1', 2') dont les axes de détection (A1, A2) forment un angle aigu (φ) par rapport à la direction de déplacement (R) du véhicule ou analogue.

3. Détecteur de collision selon la revendication 2 caractérisé en ce que les angles formés par les axes de détection (A1, A2) par rapport à la direction de déplacement (R) sont situés dans un même plan commun.

4. Détecteur de collision selon la revendication 2 ou 3 caractérisé en ce que les angles formés par les axes de détection (A1, A2) par rapport à la direction de déplacement (R) sont égaux mais de signe opposé.

5. Détecteur de collision selon l'une des revendications 1 à 4 caractérisé en ce que le circuit d'évaluation (25 à 27) des signaux de sortie du capteur d'accélération (1', 2') comporte un microprocesseur (27).

6. Détecteur de collision selon la revendication 2 caractérisé en ce que les angles formés par les axes de détection (A1, A2, ...) par rapport à la direction de déplacement (R) ne sont pas situés dans un même plan.
